# EUROPEAN PATENT APPLICATION

(11) **EP 4 417 685 A1**
(43) Date of publication of application: **21.08.2024**
(21) Application number: 23382142.0
(22) Date of filing: 15.02.2023
(51) Int. Cl.: C12N 5/00, B33Y 70/00, B33Y 80/00, C12N 5/077, C12N 5/09, G01N 33/50

(54) **BIOPRINTED THREE_DIMENSIONAL TUMOR MODEL**

(71) Applicant: Asociación Centro de Investigación Cooperativa en Biomateriales - CIC biomaGUNE, 20014 San Sebastián (ES); Administración General De La Communidad Autónoma De Euskadi, 01010 Vitoria-Gateiz (Álava) (ES)
(72) Inventor: GONZÁLEZ CALLEJO, Patricia, SAN SEBASTIÁN 20014 (ES); VÁZQUEZ ARISTIZABAL, Paula, 20014 SAN SEBASTIÁN (ES); LIZ-MARZÁN, Luis Manuel, 20014 SAN SEBASTIÁN (ES); IZETA PERMISÁN, Ander, 20005 SAN SEBASTIAN (ES); JIMENEZ DE ABERASTURI ARRANZ, Dorleta, 20014 SAN SEBASTIAN (ES); HENRIKSEN-LACEY, Malou, 20014 SAN SEBASTIÁN (ES); GARCÍA ASTRAIN, Clara, 20014 SAN SEBASTIÁN (ES)
(74) Representative: Herrero & Asociados, S.L.

(57) **Abstract**

The present invention relates a bioprinted three-dimensional tumor model which is able to reproduce the cellular heterogeneity, the spatial architecture, and the tissue-specific ECM of the tumor microenvironment. In addition, the invention refers to a process for preparing such three-dimensional tumor model, to methods for testing anticancer drugs as well as to the use of the tumor model in developing personalized medicine and developing new therapies based on the understanding of the in vitro tumor progression.

## Description

### FIELD OF THE INVENTION

The present invention belongs to the field of tumor models for drug testing and for *in vitro* research purposes. More specifically, the present invention refers to a bioprinted three-dimensional tumor model which is able to reproduce the physiopathological complexity of real tumors, mimicking the tumor microenvironment and the original tumor tissue extracellular matrix. In addition, the invention refers to a process for preparing such a three-dimensional tumor model, to methods for testing anticancer drugs, as well as to the use of the tumor model in developing personalized medicine and developing new therapies based on the understanding of the *in vitro* tumor progression.

### BACKGROUND OF THE INVENTION

Although several advances have been made regarding cancer treatment during the last decades, cancer is still in many cases an incurable disease with a high mortality rate worldwide. Cancer patients display high levels of inter-patient variation in terms of prognosis, response to therapy, and subsequent clinical outcomes. Molecular, phenotypic and functional diversity among tumors from different cancer patients, are features that can dictate therapeutic responses.

Among different cancer subtypes, breast cancer is known to be one of the most heterogeneous, representing the most prevalent disease among women worldwide, the most frequently diagnosed cancer, and the leading cause of cancer death in women. The heterogeneity of breast tumors imposes on patient's prognosis and response to therapy.

For this reason, the development of personalized preclinical studies that guarantee and determine the most appropriate therapeutic options for each patient is of great relevance. The development of a prognostic preclinical *in vitro* model capable of mimicking this diversity *ex vivo* would provide an interesting alternative to identify the most suitable treatment regimens for individual patients in a personalized manner. Unfortunately, there are few *in vitro* tumor models available to reflect tumor complexity that serve to monitor therapeutic effectiveness of pharmacological treatments for each patient. One of the main limitations is the poor reproducibility of the tumor microenvironment (TME). The TME is a highly complex niche where tumor cells are surrounded by various types of non-malignant cells, including stromal and immune cells embedded within the extracellular matrix (ECM), which plays a pivotal role in cancer initiation, progression, and therapy response. The TME is characterized by the complex bidirectional communication network that is established between cancer cells, stromal cells, and ECM components. Hence, it is essential to preserve the features of the TME when designing *in vitro* tumor models for pre-clinical drug testing. The recreation of the TME entails a challenge when developing complex 3D culture *in vitro* models methods.

Recently, 3D bioprinting has shown great potential in the realistic recreation of tissues, mainly thanks to its ability to assemble various cell types and biomaterials with high spatial resolution, thus forming bioengineered precise 3D constructs. Thanks to this technology, 3D bioprinted models can recreate the complexity of *in vivo* tumors, including the TME and their functional and structural hierarchies, with high resolution. Different attempts to develop *in vitro* tumor models have been made, such as perfusable layered models (Choi et al., 2015) and spheroid-based metastasis models (Guzman et al., 2017).

It should be stressed that, the bioprinting biomaterials (bioinks) must provide suitable micro- and macro-structure, as well as mechanical properties for cell adhesion, proliferation and differentiation. Among the various bioinks of interest used in the above described models, synthetic and natural hydrogels from non-tissue derived sources have been the gold standard materials used for rendering the 3D models.

Tissue-specific decellularized ECM (dECM), obtained by removing all cellular components from various tissue sources, can offer improved TME properties to recreate specific tumor models because they can offer the ability to recreate a specific tumor niche ECM environment, when compared against synthetic and natural hydrogels.

The development of a more personalized medicine in the treatment of tumor pathologies requires to reproduce the TME, which in itself is a difficult task. Additionally, the cellular environment surrounding the tumor and the morphology of the tumor have an impact on the therapeutic approach.

Given the ability of bioprinted 3D tumor models to replicate a patients' specific disease in an *ex vivo* setting, one of the most relevant applications of this 3D bioprinted tumor model technology would be for drug discovery and drug development and importantly in the design of pre-clinical drug screening platforms to support therapeutic decision-making. 3D bioprinting platforms can facilitate the high-throughput screening of drug testing for multiple chemotherapeutic drugs on patient-derived bioprinted cancer models, thereby identifying the most effective combination of chemotherapeutic drug candidates for each individual patient. Although publications like Sears et al., 2019, Moroni et al., 2022, or Shah et al., 2023, have reported the generation of advanced 3D printed *in vitro* breast cancer models, the architecture and the biochemical composition of the chosen materials do not reflect the complexity of the native tissues in most cases.

Now, the inventors have developed a new bioprinted three-dimensional tumor model that is capable of reproducing the ECM, the cellular heterogeneity and the spatial architecture of a patient's tumor, thus mirroring the natural tumor microenvironment *in vitro.* The 3D tumor model herein provided is highly and easily reproducible, which renders it a powerful research tool in tumor and cancer research, which can be exploited for the development of high throughput drug screening platforms.

### BRIEF DESCRIPTION OF THE FIGURES

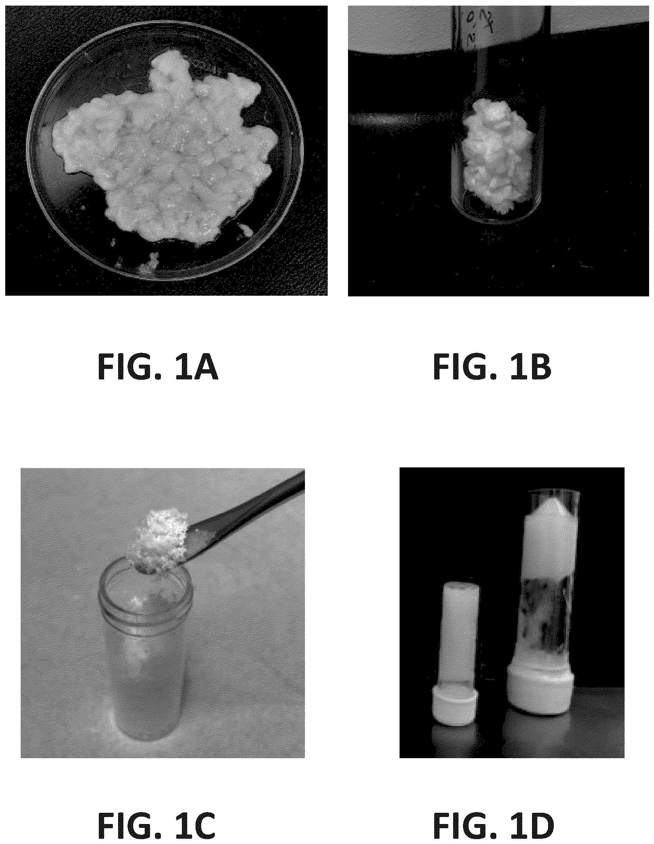

**Figure 1****:** represents macroscopic images of the obtention of the dissected breast tissue (A), freeze-dried decellularized ECM (B), dECM powder (C) and dECM ink (D).

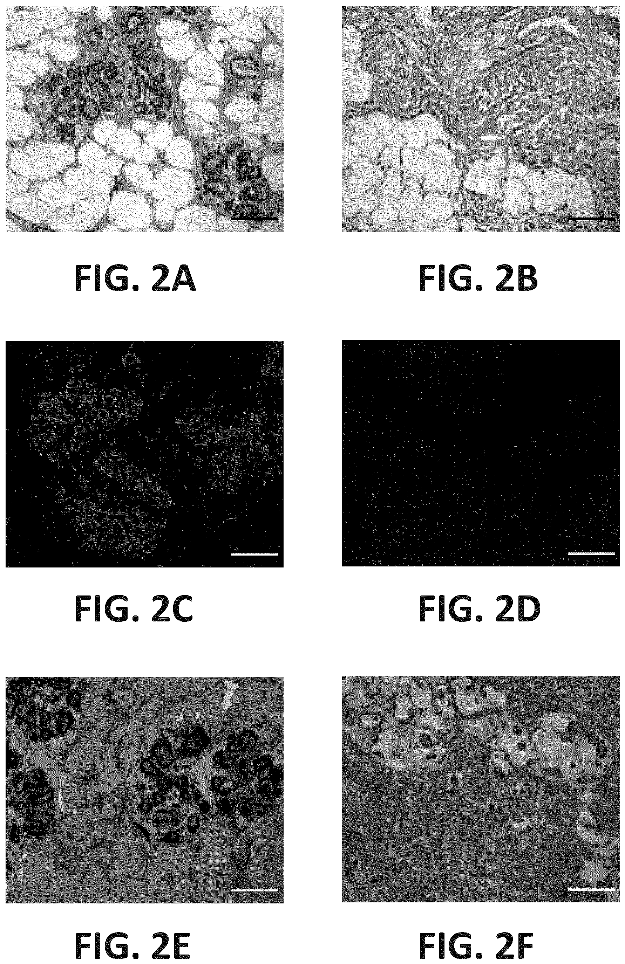

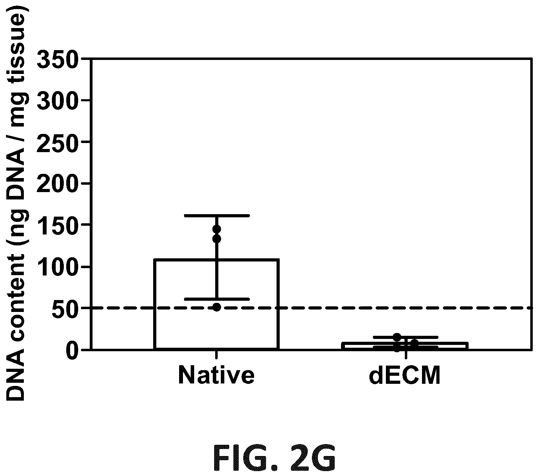

**Figure 2****:** shows H&E-stained sections of native and decellularized porcine breast tissue (A-B). DAPI-stained sections of the native and decellularized breast tissue (C-D). Oil Red O-stained sections of the native and decellularized breast tissue (E-F). Scale bar: 100 µm. Resident and remnant DNA amount quantification (G).

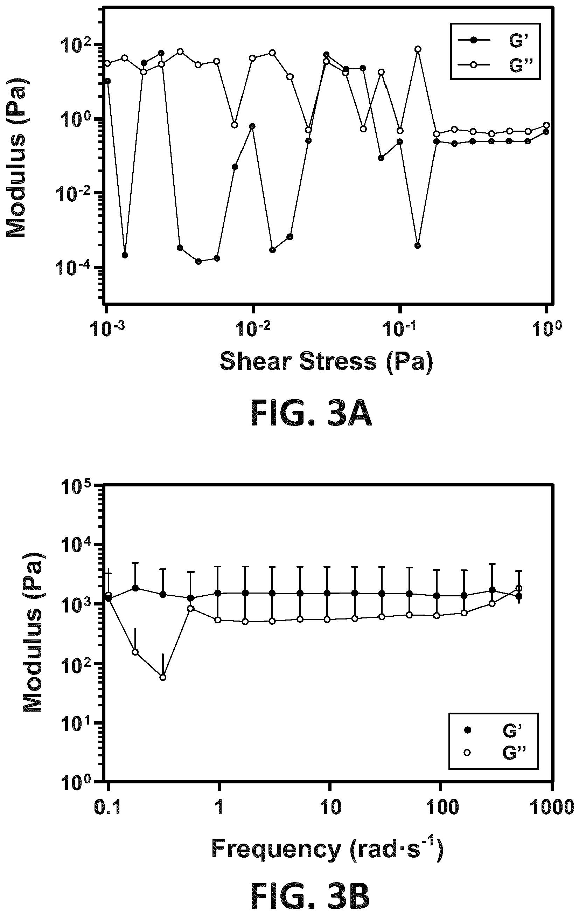

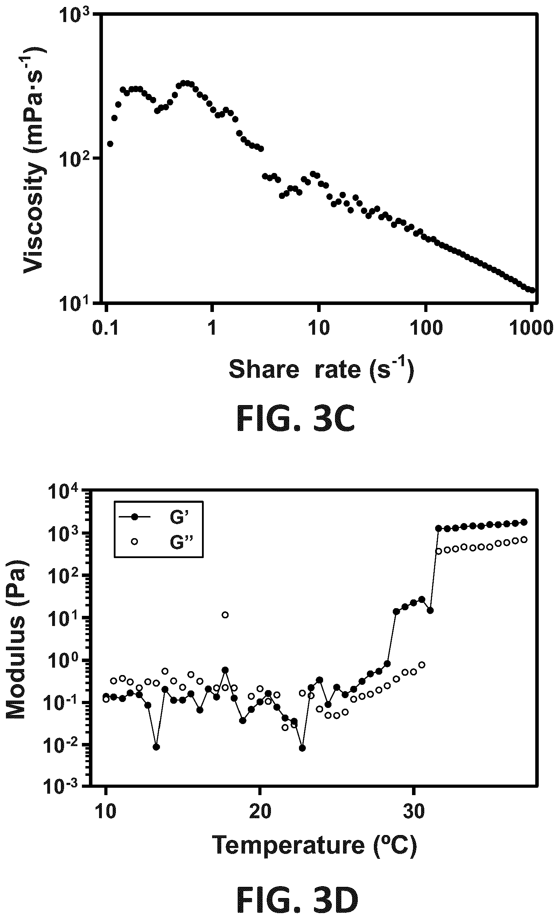

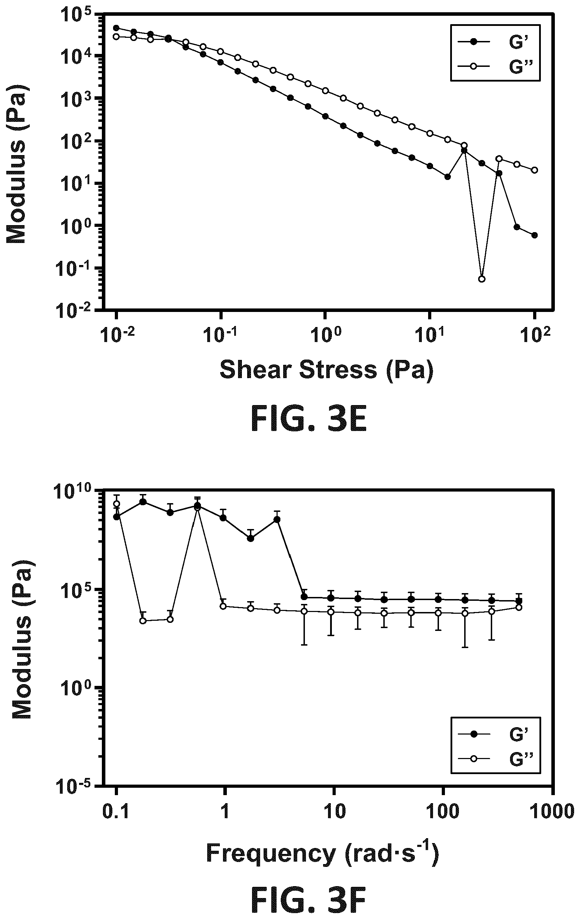

**Figure 3****:** shows the rheology of the breast 2% dECM. Amplitude sweep (A), frequency sweep (B), viscosity curve (C) and temperature ramp (D) of the ink. Modulus evaluation by amplitude sweep (E) and frequency (F) of the cross-linked gels.

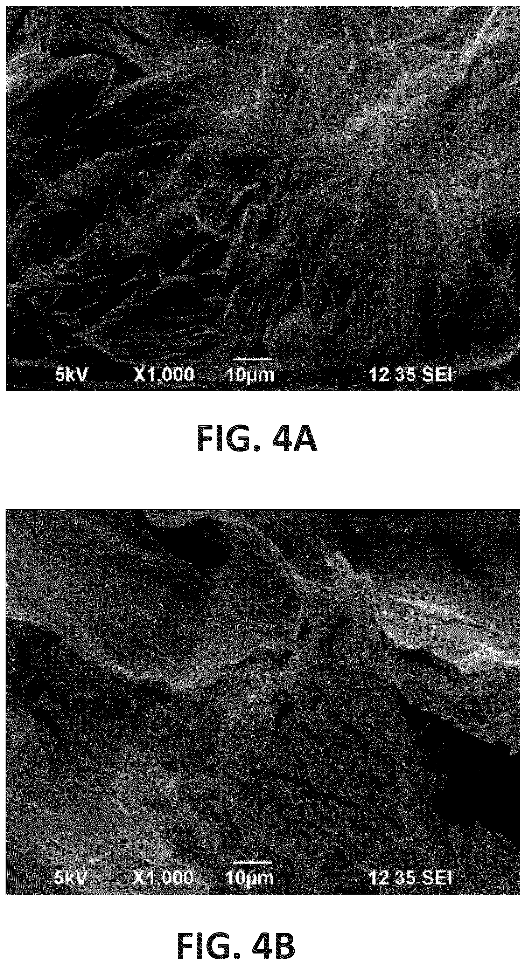

**Figure 4****:** demonstrates the scanning electron microscopy images of the top (A) and cross-section (B) of the breast dECM gel.

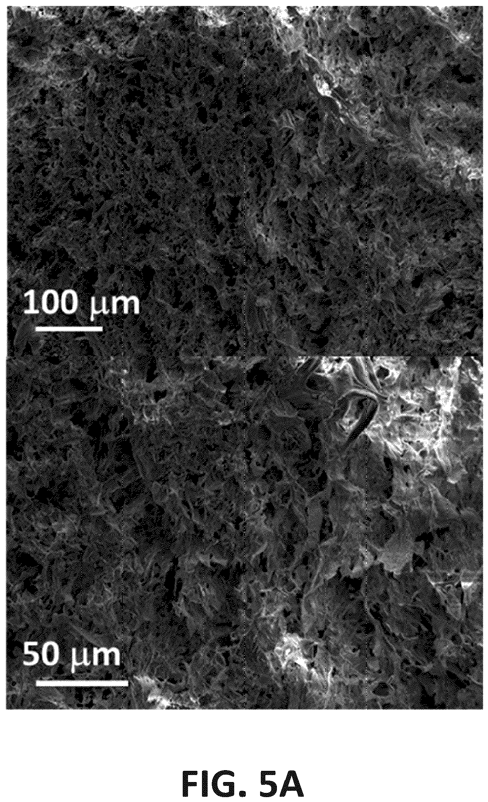

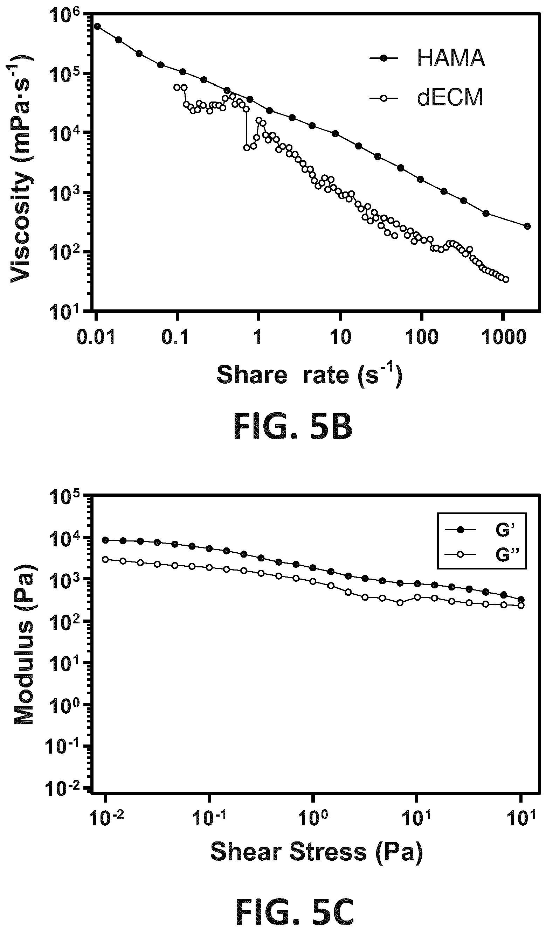

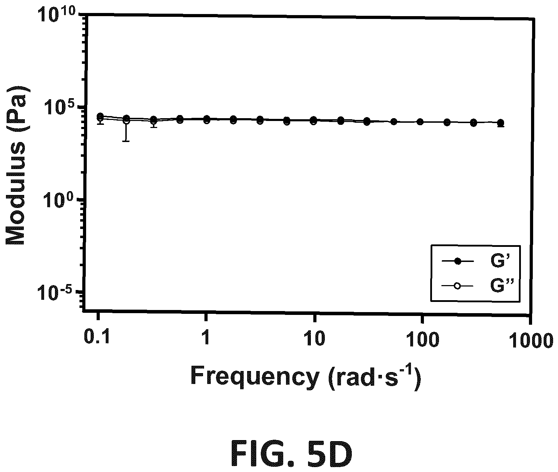

**Figure 5****:** demonstrates the scanning electron microscopy images of the cross-section of the breast dECM gel mixed with 2% (w/v) methacrylated hyaluronic acid (HAMA) (A), the viscosity curve of the breast dECM compared to the breast dECM mixed with HAMA (B), the amplitude sweep of the cross-linked mix of 2% (w/v) breast dECM with 2% (w/v) HAMA (C) and its frequency sweep (D).

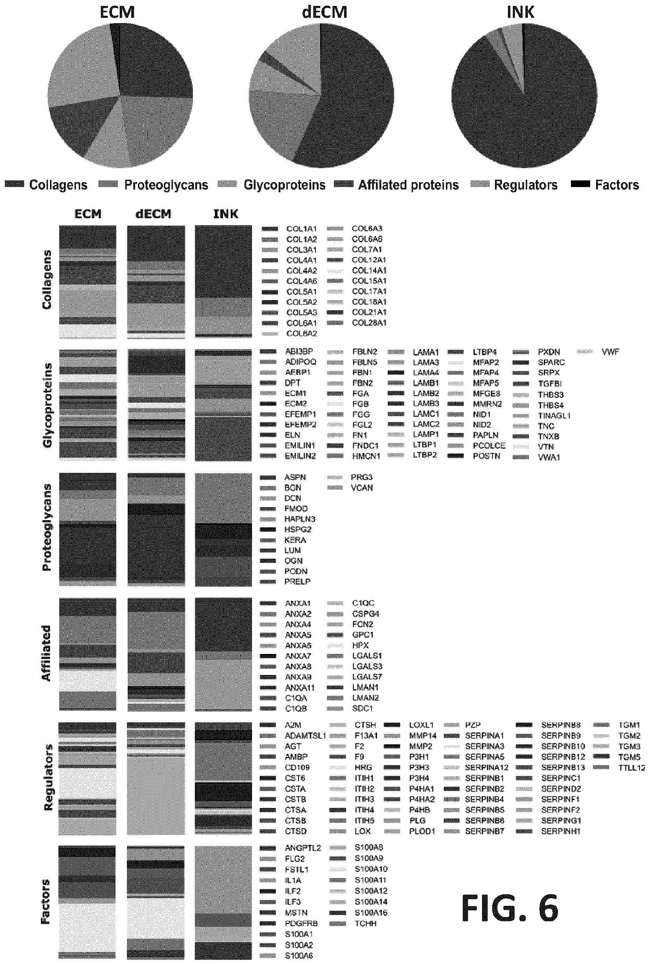

**Figure 6****:** shows matrisome of the native ECM, dECM and ink including collagens, glycoproteins, proteoglycans, affiliated proteins, regulators and secreted factors.

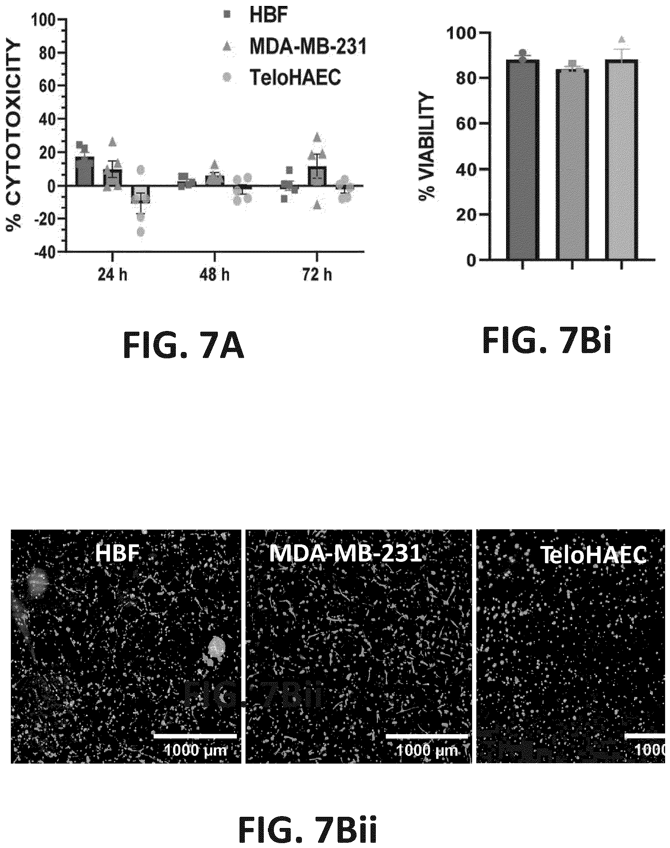

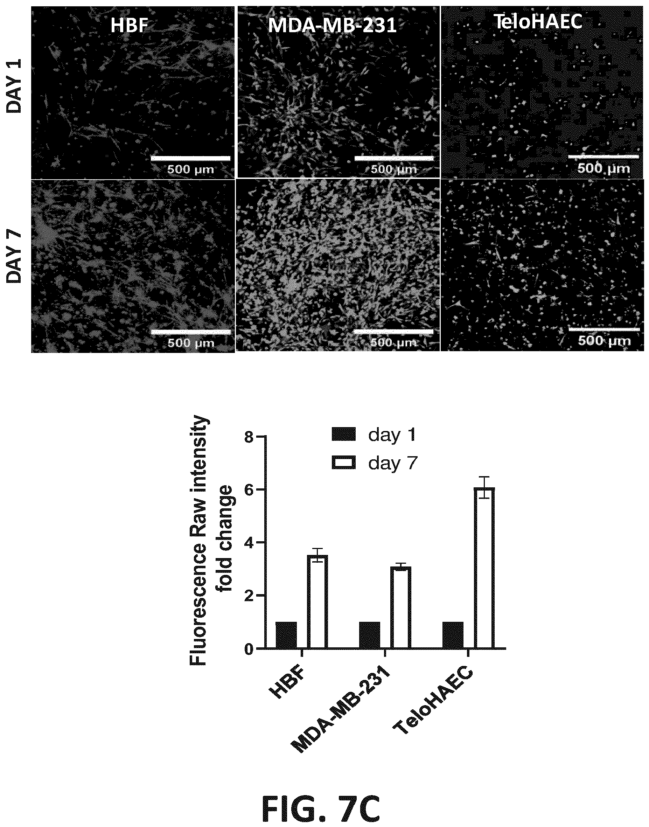

**Figure 7****:** indicates the biocompatibility assessment of Human breast fibroblasts (HBF), MDA-MB-231 cancer cells and TeloHAEC Endothelial cells incubated with breast dECM ink by cell death evaluation through LDH release assay (A), the viability assay by live (green) and dead (red) staining assay (B). Cell proliferation of cells embedded in the breast dECM in depicted in figure 7C.

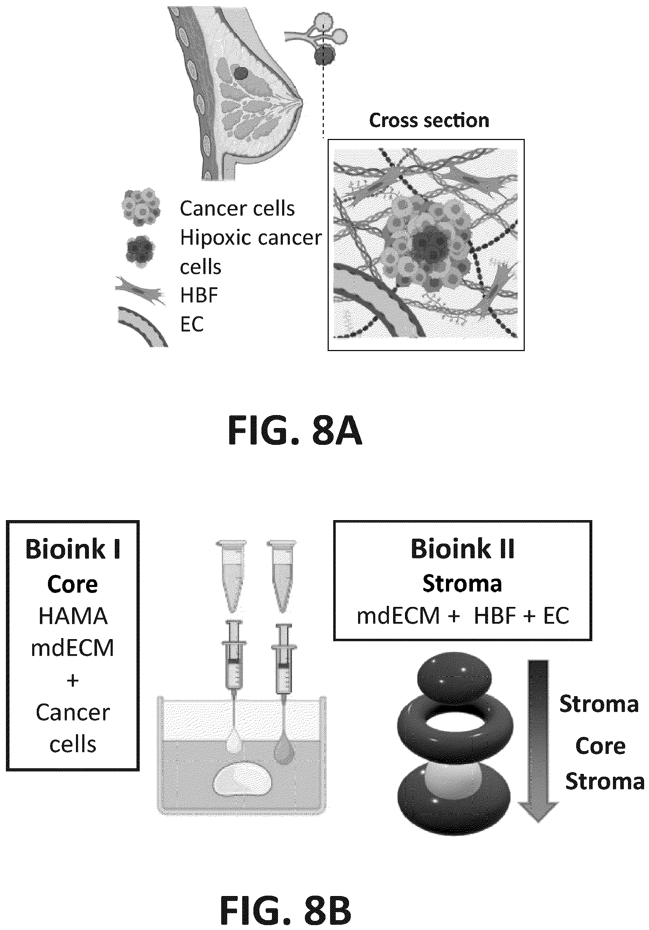

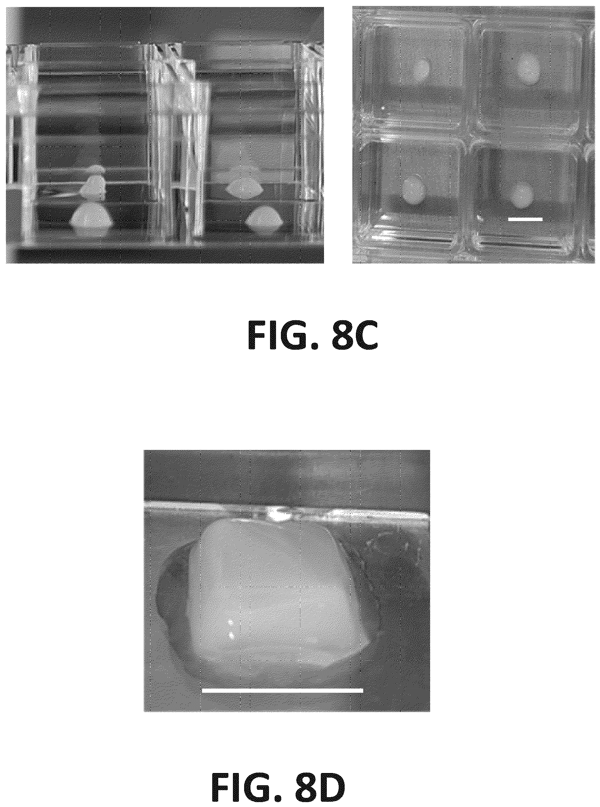

**Figure 8****:** shows a representative illustration of the breast tumor microenvironment (A). FIG 8B shows a non-limiting example of a (schematic and photographic) diagram depicting the construction of a bioprinted tumor construct, in this case, a breast tumor 3D model. The construct is obtained by a design of a four-layered circle with an inner region of tumor cells surrounded by an outer region of fibroblasts and endothelial cells. In this case, a tumor model with a tumor tissue core including breast cancer cells surrounded by stromal tissue including normal human mammary fibroblasts and artery derived endothelial cells. Examples of bioprinted tumor core are detailed in (C) and an example of the 3D tumor model with the stromal compartment is depicted in (D).

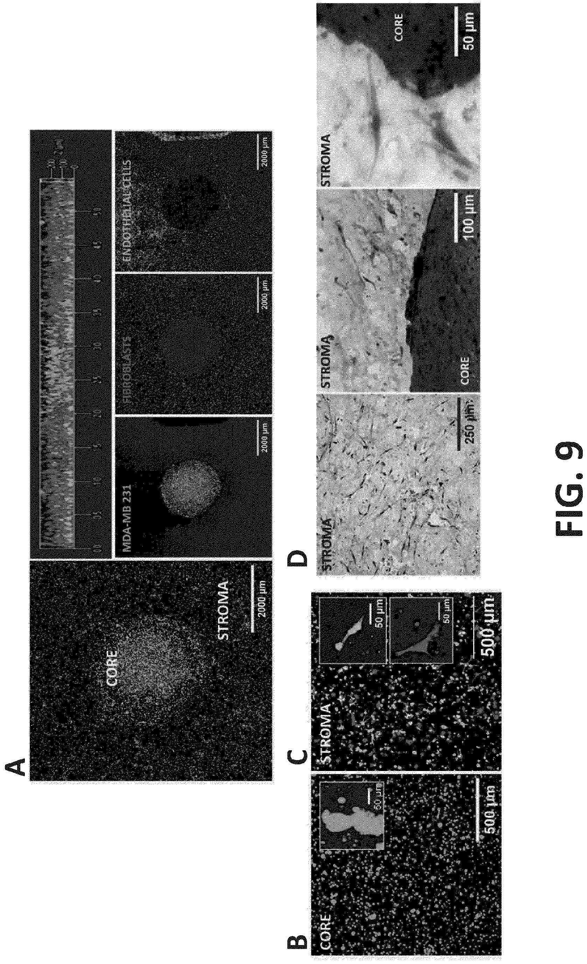

**Figure 9****:** shows a representative example of the bioprinted construct of FIG. 8 in this case, live confocal images depicting fluorescently stained cell specimens to visualize their relative positions in the bioprinted 3D architecture (A), in this case the core of labelled breast tumor cells (green) (B) and the stromal fibroblasts (pink) and endothelial cells (cyan) (C). Hematoxylin-Eosin staining images of the stroma and the core of the model are depicted in D.

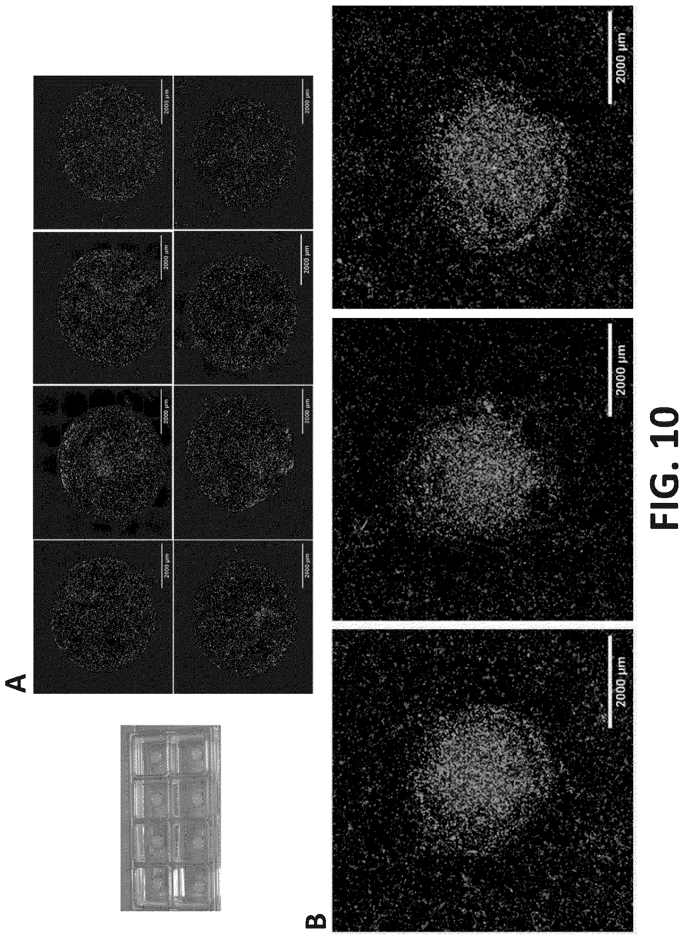

**Figure 10****:** shows a non-limiting series of representative examples of the bioprinted core in dECM enriched in HAMA in an 8-well-plate and representative confocal images of the tumor cells forming the core structure (A). FIG. 10B indicates the model reproducibility, as it shows a non-limiting series of representative examples of the bioprinted construct of FIG. 8; in this case, confocal images depicting fluorescently stained cell specimens, in this case the core of labelled breast tumor cells (green) and the stromal fibroblasts (pink) and endothelial cells (cyan) (B).

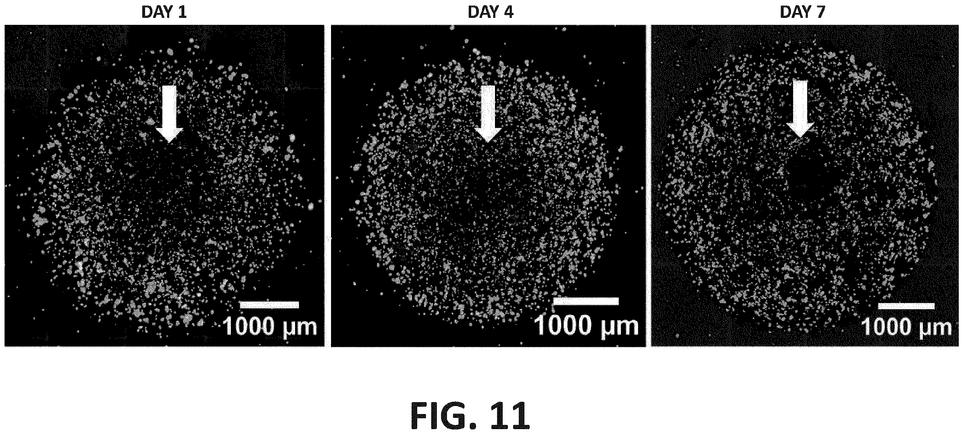

**Figure 11****:** shows examples of the cell viability of the bioprinted core of tumor cells over time, in this case at days 1, 4 and 7 after printing in which live cells are stained with calcein (green) and dead cells with propidium red (red).

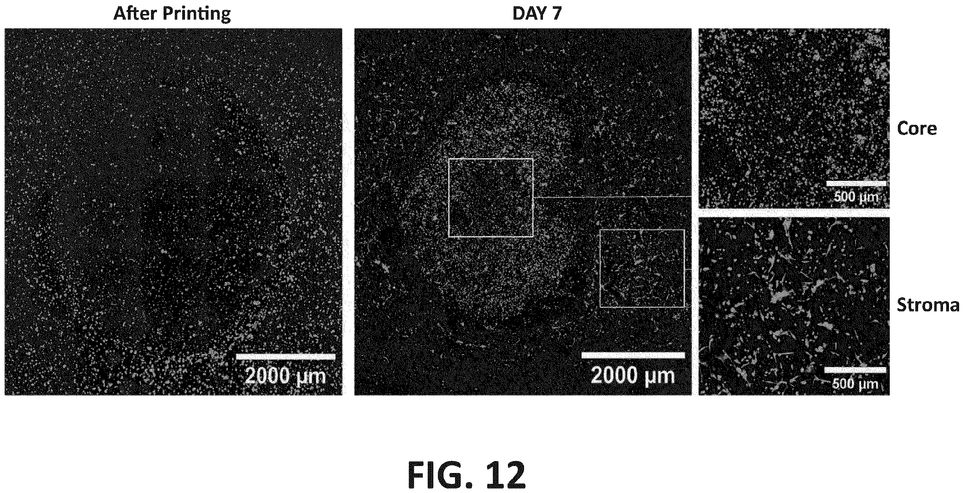

**Figure 12****:** shows examples of the cell viability in the bioprinted 3D tumor over time, in this case at days 0 and 7 after printing in which live cells are stained with calcein (green) and dead cells with propidium red (red).

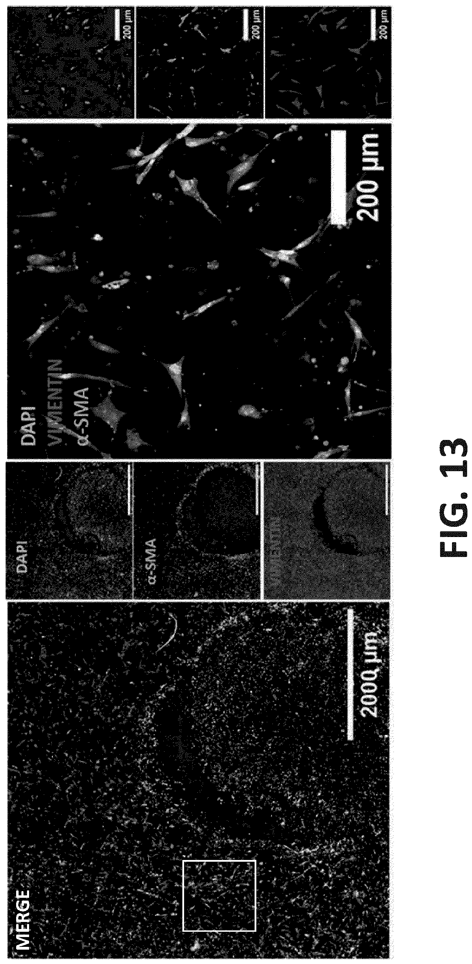

**Figure 13****:** shows a representative photomicrograph of the construct of FIG. 8 (day 7 post-fabrication); in this case, photomicrograph depicting a representative construct stained to visualize the activation of cancer associated fibroblasts by antibodies against vimentin (pink) and α-SMA (green), as well as DAPI (cyan).

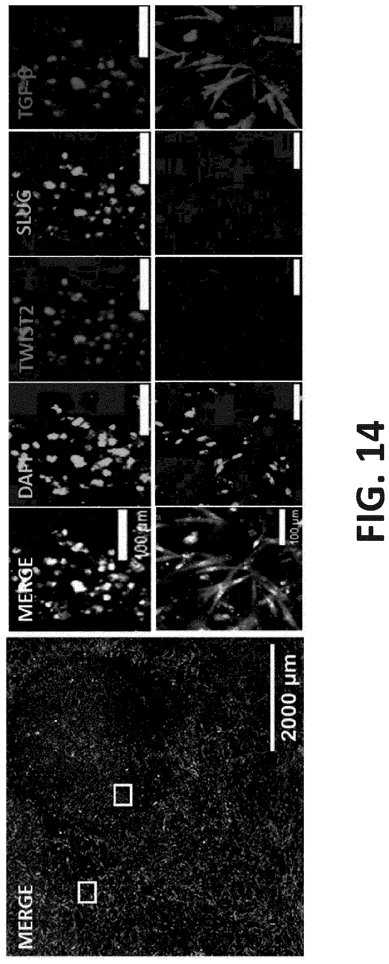

**Figure 14****:** shows a representative photomicrograph of the construct of FIG. 8 (day 7 post-fabrication); in this case, photomicrograph depicting a representative construct stained to visualize the activation of tumoral biomarkers in the 3D construct by antibodies against TGF-β (pink), SLUG (green) and TWIST2 (red) as well as DAPI (cyan).

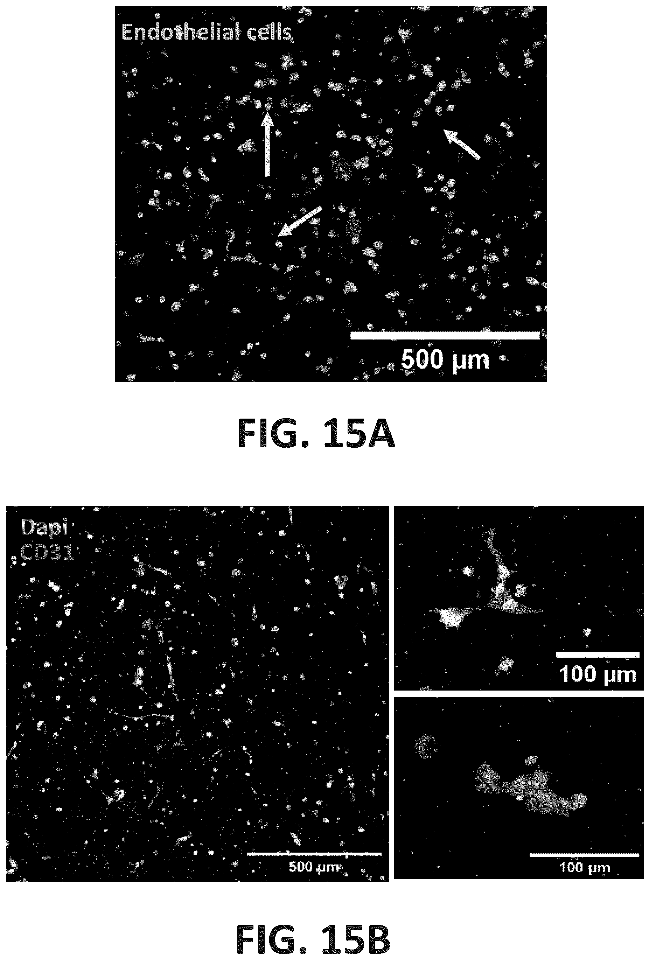

**Figure 15****:** shows specific areas of constructs stained to visualize angiogenic formation by endothelial cells by antibodies against CD31 (pink), as well as DAPI (cyan).

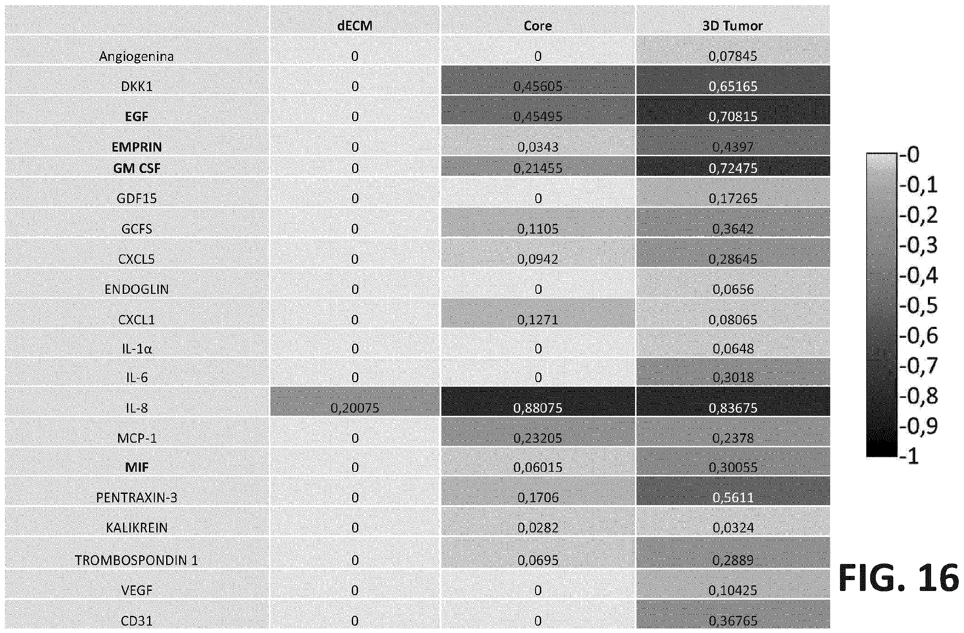

**Figure 16****:** shows the cytokine secretion pattern of the breast dECM, the tumor core and the 3D tumor model after 7 days in culture.

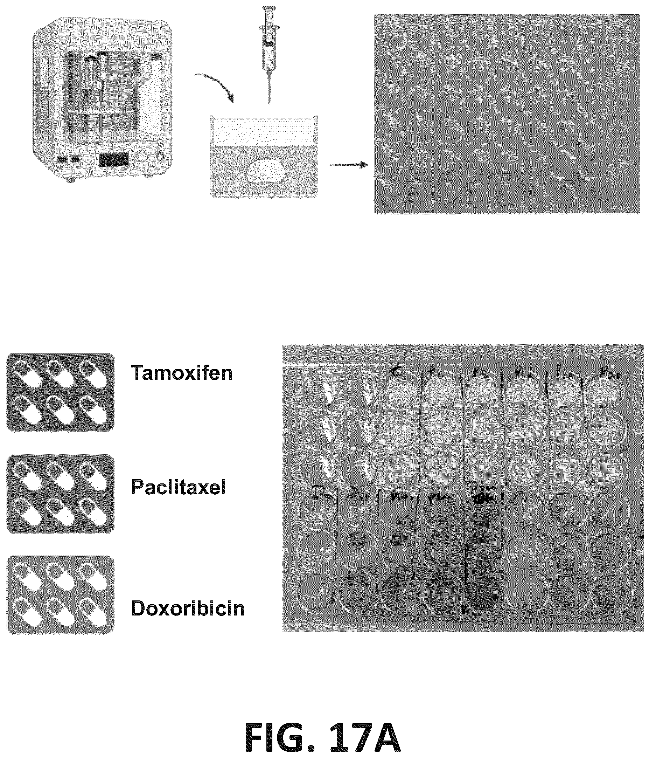

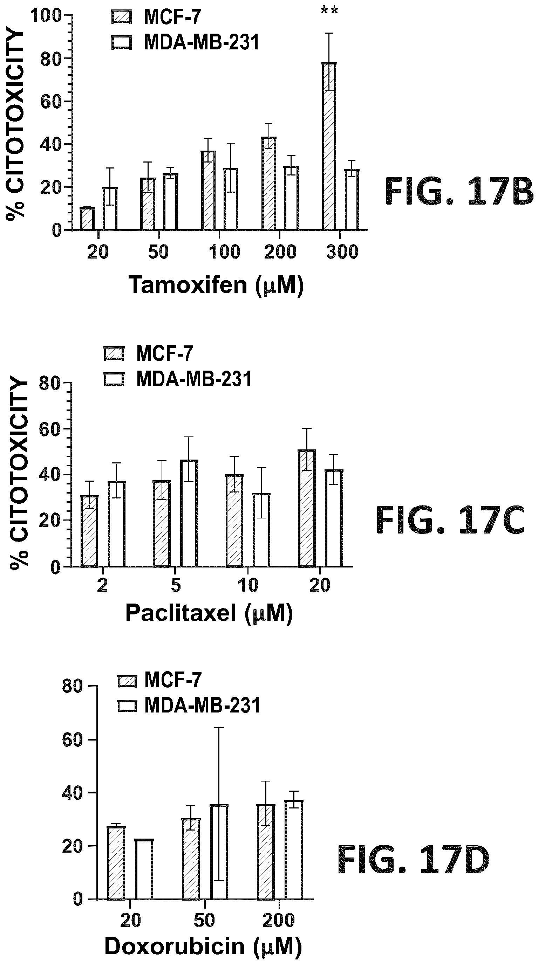

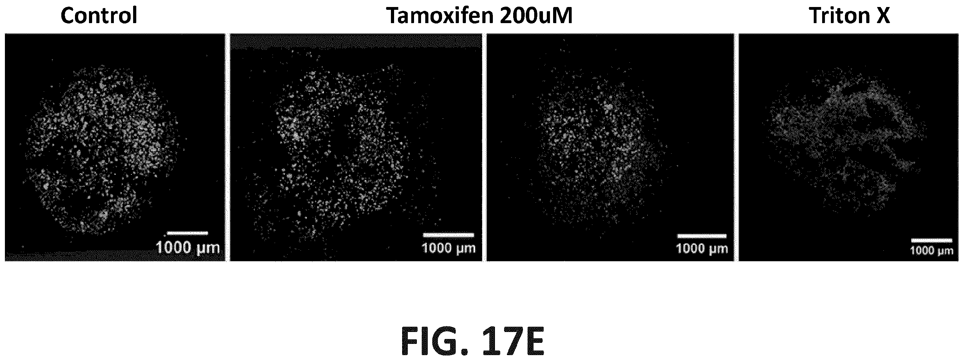

**Figure 17****:** indicates the utility of the model to be used as a high-throughput drug testing platform (A) and shows non-limiting examples of graphs depicting drug response assays to increasing concentrations of tamoxifen (B), paclitaxel (C) and doxorubicin (D) of the MCF-7 and MDA-MB-231 tumor models of FIG.8. Images of a live/dead staining of MCF-7 3D tumor models after tamoxifen treatment are depicted in (E).

### OBJECT OF THE INVENTION

The main object of the present invention is represented by bioprinted three-dimensional tumor model comprising:
a) an inner core comprising tumor cells; and
b) an outer stromal shell comprising fibroblast and/or endothelial cells surrounding the core
wherein the cells from the core and the stromal shell are embedded in a decellularized extracellular matrix (dECM).

It is also an object of the invention process to prepare a three-dimensional tumor model according to the invention.

Further, it is an object of the invention a method for testing anticancer drugs based on the use of a three-dimensional tumor model according to the invention.

Finally, it is an object of the invention the use of the bioprinted three-dimensional tumor model according to the invention to reproduce the physiopathological complexity of the tumor microenvironment, to develop a personalized medicine, and for developing new therapies based on the understanding of the tumor progression in a three-dimensional environment mimicking the tumor microenvironment.

### DETAILED DESCRIPTION OF THE INVENTION

The following definitions are provided to assist in understanding and interpreting the present invention:
**"Three-dimensional tumor model" or "3D tumor model":** it refers to an *in vitro* reproduction in three dimensions of a certain tumor, produced by bioprinting. It represents the main object of the invention, and it is formed by two differentiated structures: a) an inner core comprising or consisting essentially of tumor cells within a dECM optionally enriched with an additive, preferably HAMA and b) an outer stromal shell surrounding the inner core comprising or consisting essentially of fibroblasts and/or endothelial cells within a dECM optionally enriched with an additive. Although the basic model is as explained, the inner core and the outer shell may comprise further types of cells, especially immune cells for reproducing situations that may occur during the progression of a tumor. In the preferred embodiment of the invention, the three-dimensional tumor model of the invention is a 3D breast tumor model.
**"Bioink":** it refers to any formulation used in bioprinting technologies, usually consisting of a biocompatible material and a compulsory cellular component.
**"Bioprinted" or "bioprinting:** it refers to the way or technique for producing the 3D tumor model of the invention, which is based on the use of bioinks comprising or consisting essentially of a decellularized matrix (dECM) fraction and a cellular fraction containing the cells. Both the composition of the dECM fraction and of the cellular fraction of the bioink depend on whether the bioink is intended for the bioprinting of the inner core or of the outer stromal shell. In the context of the invention, any bioprinting machine or apparatus is suitable for producing the 3D tumor model of the invention, as long as it is adapted for bioprinting the bioinks.
**"Inner core":** it refers to the internal part of the three-dimensional tumor model of the invention, which comprises or consists essentially of a dECM preferably enriched in HAMA and tumor cells. The inner core may optionally contain other types of cells such as for instance immune cells.
**"Stromal Shell":** it refers to the external part of the three-dimensional tumor model of the invention surrounding the inner core and comprises or consists essentially of a dECM, optionally including at least one additive and fibroblasts and/or endothelial cells. The outer stromal shell may optionally contain other types of cells such as for instance immune cells.
**"Decellullarized extracellular matrix"** or **"dECM":** in the context of the invention, it refers to the substance in which the cells of both the inner core and the stromal shell are embedded and which gives structure and support to the cells, as well as allowing them to grow three-dimensionally in the 3D tumor model of the invention. The dECM is composed mainly of collagens and other types of proteins, as well as of regulators and other bioactive molecules. The dECM may be standard, commercially available dECMs, such as for instance Matrigel^{®}, rat tail collagen type I, or any other similar product, or more preferably it is obtained from the same tissue of origin of the particular tumor model. The use of dECM derived from the tissue of origin of the tumor ensures the presence of ECM proteins and bioactive molecules found in the normal microenvironment of the tumor.
**"Tumor cells":** in the context of the invention, it refers to the cells present in the inner core of the 3D tumor model. They can generally be understood as malignant cancer cells that divide and proliferate in an incontrollable manner. Any type of tumor cells may be used in the context of the invention but preferably the tumor cells are derived from a patient's tumor or selected from different subtypes of breast tumors, preferably MDA-MB-231, MCF-7 and HCC1806 cell lines, pancreatic tumors, preferably MIA Paca2 and Capan-2 cell lines, lung tumors, preferably H69PR and A549 cell lines, kidney tumors, preferably A-498 and 786-0 cell lines, hepatic tumors, preferably HepG2 and HepT1 cell lines, prostate tumors preferably DU145, PC3, and LNCaP cell lines, ovarian tumors, preferably SK-OV-3, A2780 and OVCAR-3 cell lines.
**"Fibroblasts":** in the context of the invention, it refers to a type of cells present in the outer stromal shell of the 3D tumor model. It is the type of cells that synthesize the extracellular matrix and collagen, produce the structural framework (stroma) for animal tissues, and play a critical role in wound healing. Fibroblasts are the most common cells of connective tissue in animals. Any fibroblast may be used in the context of the invention but preferably fibroblasts are derived from the same source as the tumor cell, i.e. from the own patient. It is also preferred that fibroblasts are derived from the same tissue of origin of the tumor.
**"Endothelial cells":** in the context of the invention, it refers to a type of cells present in the outer stromal shell of the 3D tumor model. It is the type of flat cell that forms the inside of all blood vessels (including capillaries) and is in permanent contact with blood. They act as regulators of cellular and molecular traffic from blood into tissues. Any endothelial cell may be used in the context of the invention but preferably endothelial cells are derived from the same source as the tumor cells, i.e. from the own patient, but also commonly known and established endothelial and fibroblast cell lines may be used, for instance, endothelial HUVEC or HAEC cell lines.
**"Immune cells":** in the context of the invention, it refers to different types of cells that may be present in some embodiments, either in the inner core or in the stromal shell, or in both, within the 3D tumor model. It generally refers to any type of cells from the innate or adaptative immune system that help in the protection of organisms from infections and diseases, including cancer. Any type of immune cells may be used in the model of the invention without limitation but in a preferred embodiment immune cells comprise macrophages, natural killer cells, dendritic cells and T lymphocytes.
**"Tumor derived factors":** generally refers to substances produced by cancer cells that can influence the behavior of surrounding normal cells, blood vessels, and immune cells. These can include cytokines, growth factors, enzymes, and other molecules that affect the tumor microenvironment and contribute to tumor growth, progression, and metastasis. These factors can also influence the behavior of normal cells in the area, such as blood vessels, immune cells, and/or fibroblasts, and modulate the host response to the tumor.
**"Enriched with methacrylated hyaluronic acid (HAMA)":** in the context of the invention, it refers to adding HAMA to the dECM during the preparation of the bioink for bioprinting, with the aim of modifying the mechanical properties of the resulting bioprinted product. Although both the first and the second bioink may be enriched with HAMA, a preferred embodiment is that where only the first bioink is enriched with HAMA.
**"Tumor microenvironment or "TME":** it generally refers to all the components and conditions surrounding a tumor *in vivo,* from physical, physiological, biochemical, cellular, and mechanical points of view. The 3D tumor model of the invention mimics the tumor microenvironment.
**"Regular morphology":** it refers to those embodiments where the inner core of the 3D model of the invention has essentially a geometric shape, preferably a sphere or spheroid. The term spheroid refers to the regular morphology when the geometric shape most closely related to the regular morphology is a sphere.
**"Irregular morphology":** it refers to those embodiments where the inner core of the 3D model of the invention has an amorphous or essentially non-geometric shape that can nevertheless be reproduced by means of the bioprinting technology.
**"Maximum diameter":** it refers to the maximum distance from one end-point of the inner core to an opposite end-point.
**"First Bioink":** in the context of the invention, it refers to the bioink used to prepare the inner core of the 3D tumor model of the invention. Its basic composition comprises the mixture of the tumor cells with dECM and optionally other cells such as immune cells or other additives such as HAMA. In a preferred embodiment, the first bioink comprises tumor cells and dECM enriched in HAMA.
**"Second Bioink":** in the context of the invention, it refers to the bioink used to prepare the outer stromal shell of the 3D model of the invention. Its basic composition comprises the mixture of fibroblasts and/or endothelial cells with dECM, and optionally other cells such as immune cells or other additives such as HAMA. In a preferred embodiment, the second bioink comprises fibroblasts, endothelial cells and dECM.
**"Tissue of origin"** or **"tissue of origin of the tumor":** it refers to the the biological tissue where the tumor grows in the patient. This expression is applicable to those embodiments where the dECM is obtained from the same tissue where the tumor object of the particular model developed was originated. *Mutatis mutandis* it can also refer to the tissue of origin of the fibroblasts, endothelial cells and/or immune cells.
**"Tumor models of different nature":** in the context of the present invention, it may refer to tumor model developed from different patients, or to tumor models of different type of tumors, such as for instance, breast tumor and pancreatic tumor or even to tumor models from different subtypes of the same type of tumor such as, for instance, HER2-enriched, triple negative, luminal a or luminal b breast cancer.
**"High-throughput testing or "high-throughput screening (HTS)":** in the context of the invention, it refers to a method used in drug discovery to rapidly test the biological activity of large numbers of compounds using automated equipment and techniques. The goal of HTS is to identify potential drug candidates from a large library of compounds and prioritize them for further testing and development. This approach allows for efficient screening of many compounds in a short period of time, leading to faster and more cost-effective drug discovery.

### Bioprinted three-dimensional tumor model

In a first aspect, the invention refers to a bioprinted three-dimensional tumor model comprising:
a) an inner core comprising tumor cells; and
b) an outer stromal shell comprising fibroblasts and/or endothelial cells surrounding the core;
wherein the cells from the core and the stromal shell are embedded in a decellularized extracellular matrix (dECM).

This bioprinted three-dimensional tumor model can be referred to indistinctly along the present specification as "bioprinted tumor model of the invention", "bioprinted model of the invention", the "three-dimensional tumor model of the invention", "three-dimensional model of the invention", "tumor model of the invention" or simply "model of the invention".

The model of the invention is a 3D structure with two clearly differentiated parts, namely an inner core and an outer stromal shell.

The first one is the inner core and comprises or consists essentially of tumor cells embedded in a dECM. In a preferred embodiment of the invention the dECM used for the bioprinting of the inner core is enriched with HAMA. HAMA used in the dECM for the preparation of the inner core has the effect of providing a stiffer matrix, giving the model of the invention mechanical properties similar to those observed in real tumors. Several rheological modifiers such as methacrylated gelatin, gelatin, hyaluronic acid, or collagen, can also be added to the dECM to improve the quality of the bioprinting.

The type of tumor model will mainly depend on the type of tumor cells used for the preparation of the model of the invention. For instance, a breast tumor model can be developed according to the invention by using breast tumor cells in the preparation of the inner core.

In the preferred embodiment of the invention, the tumor cells are derived from a patient's tumor such that the model allows to assess and develop a specific and personalized therapy against the particular patient's tumor.

Although any type of tumor model may be prepared, in a particular embodiment of the invention, the tumor cells are either cells derived from a patient's tumor or commercial cells selected from different subtypes of breast tumors, preferably MDA-MB-231, MCF-7 and HCC1806 cell lines; pancreatic tumors, preferably MIA Paca2 and Capan-2 cell lines; lung tumors, preferably H69PR and A549 cell lines; kidney tumors, preferably A-498 and 786-0 cell lines; hepatic tumors, preferably HepG2 and HepT1 cell lines; prostate tumors, preferably DU145, PC3, and LNCaP cell lines; and ovarian tumors, preferably SK-OV-3, A2780 and OVCAR-3 cell lines.

In a preferred embodiment of the invention, the tumor model of the invention is a breast tumor model based on the use of breast tumor cells, preferably derived from a patient suffering from breast cancer.

The second structurally differentiated part of the 3D model of the invention is the outer stromal shell, which comprises or consists essentially of fibroblasts and/or endothelial cells embedded in a dECM which completely surrounds the inner core.

From an anatomical point of view, stroma is formed by the cells and structures giving support to organs, glands and tissues. Although small differences exist in the stroma depending on their exact origin, all stromas have in common the presence of fibroblast forming the connective tissue and endothelial cells forming the vessels that perfuse the tissues and organs. With the intention of replicating the natural conditions surrounding tumors, the stromal shell in the model of the invention comprises or consists essentially of fibroblasts and/or endothelial cells embedded in a dECM. The presence of fibroblasts and/or endothelial cells together with the dECM reproduce the natural microenviroment of the tumor in the model of the invention.

Ideally, fibroblasts and endothelial cells isolated from the patient having the tumor to be tested are present in a preferred embodiment of the invention, however, also commonly known and established endothelial and fibroblasts cell lines may be used. Endothelial cell lines emulating tumoral vascularization will be maintained in the recreation of any stroma subtypes. For instance, endothelial HUVEC or HAEC cell lines can be used in the model of the invention.

Regarding the fibroblastic population, the election of the stromal host tissue cells can vary depending on the tumor subtype recreation. For example, in the case of the recreation of a lung tumor, human lung fibroblasts and lung alveolar epithelial cells (HPAEpiC) can be used. In the case of recreation of a pancreatic tumor, stromal cells will consist of human pancreatic fibroblasts and pancreatic stellate cells (HPaSteC). In the case of a hepatic tumor, hepatic fibroblasts and hepatic stellate cells (HSCs) can be used. In the case of a prostate tumor, resident prostate smooth muscle cells and prostate derived fibroblasts will be used.

The dECM and the nature thereof is also a very relevant element of the invention. It has various functions. The first one is to serve as support and as a niche for the 3D growth of the cells. On the other hand, it allows reproducing the functional, mechanical, physiological, and biochemical microenvironment of the tumor.

The dECM used may be standard, commercially available dECMs, such as, for instance Matrigel^{®}, rat tail collagen type I, or any other similar product. However, such kinds of commercial dECMs are less desirable as they do not exactly reproduce the natural microenvironment of the particular and specific tumor model prepared. For that reason, in a preferred embodiment, the dECM is derived from the tissue of origin of the specific tumor model designed. For instance, if a breast tumor model is to be prepared, it is preferred that the used dECM is derived from breast tissue. The fact of using dECM derived from the tissue of origin of the tumor ensures the presence of ECM proteins and bioactive molecules found in the normal microenvironment of the tumor. Figure 6 shows the similarity in terms of composition of the dECM when compared to a natural ECM.

In addition, the dECMs may be prepared from tissues of many mammalian species, however, in a preferred embodiment the tissue used for preparing the dECM is porcine tissue. On the one hand, porcine tissue is very similar to human from a physiological and biochemical point of view, and, on the other hand, it ensures that the dECM is absent from tumor derived factors, which could be present if the patient tissue is used to prepare the dECM. It is desirable that the dECM is absent from tumor derived factors because they can activate specific oncogenic signaling that dictates genotypic and phenotypic changes in recipient cells embedded in the dECM that do not correspond to intrinsic nature of the tumor cells under study, thus hijacking the potential aggressiveness of tumor cells. In the case of a breast tumor model, the dECM can also be prepared from wastes of human breast tissue resulting from surgery, such as breast reduction or other.

The dECM used in preparing the tumor model can be modified with additives to change the mechanical properties of the tumor model. For instance, as explained above, the dECM can be enriched with HAMA to provide additional stiffness to the dECM. In the model of the invention, the dECM used for preparing the bioink for bioprinting of the inner core is preferably enriched with HAMA to provide the model of the invention mechanical properties similar to those observed in tumors *in vivo.*

Other additives that may be used to modify the properties of the model are hyaluronic acid, methacrylated gelatin, or collagen. The addition of those additives can increase the printability potential of the dECM and are components of the ECM usually found in tumor tissues.

From the morphological point of view, the bioprinted three-dimensional tumor model of the invention may have a regular or irregular morphology. In a particular embodiment the inner core has a regular shape such as a sphere or spheroid. However, natural tumors may have different shapes. The bioprinting technology allows the production of inner cores of the tumor model having any regular or irregular shape, thus reproducing the specific shape of a patient's tumor.

In terms of dimensions, the model of the invention has an inner core with a maximum diameter of 1 mm to 4 mm, preferably from 1.5 mm to 3.5 mm, more preferably 1 to 3 mm. The complete three-dimensional tumor model has a volume in the range of 6 mm³ to 1 cm³, more preferably from 0.3 cm³ to 1 cm³.

Apart from the essential elements conforming the basic model of the present invention, both the inner core and the stromal shell may contain additionally immune cells for *in vitro* mimicking naturally occurring situations. Immune cells play an important role in tumor progression and the presence thereof may reproduce different situations of early or advanced stages of a tumor. In early stages of cancer progression, immune cells are capable of mediating anti-tumor immune response. However, tumor cells acquire the ability to hijack the immune system and favor immune escape, creating an immunosuppressive microenvironment that promotes tumor growth.

In a particular embodiment of the invention, the inner core and the stromal shell may contain an immune cell component. Immune cells may comprise macrophages, natural killer cells, dendritic cells, and/or T lymphocytes. The incorporation of immune cell subsets to the model might allow the study of the immunosuppression process associated with tumor progression at different stages.

### Process for preparing a bioprinted three-dimensional tumor model

A further aspect of the present invention is a process to prepare the three-dimensional tumor model of the invention comprising:
a) the preparation of a first bioink comprising mixing at least the tumor cells with dECM, optionally enriched with HAMA;
b) the preparation of a second bioink comprising mixing at least the fibroblasts and/or endothelial cells and dECM;
c) the three-dimensional printing of the inner core with the first bioink, optionally followed by the irradiation with UV light when the dECM is enriched in HAMA; and
d) the three-dimensional printing of the outer stromal shell with the second bioink around and completely surrounding the inner core followed by incubation of the resulting tumor model at a temperature between 25 and 40 °C.

This general process for the preparation of three-dimensional tumor models can be referred to along the description as the process of the invention.

The process of the invention comprises steps directed to prepare the bioinks that are used in the bioprinting (steps a and b), as well as steps directed to the bioprinting itself of the different parts of the tumor model (steps c and d). The process for preparing the model of the invention is represented in the scheme of figure 8.

Two separate bioinks must be prepared to produce the inner core and the outer stromal shell. Bioinks are made by a mixture of the dECM optionally enriched with the additive of choice, such as, for instance HAMA, together with the specific cell types, depending on the part of the tumor model that is to be printed with such bioink.

As explained above, the used dECM may be a standard commercially available dECM, such as, for instance Matrigel^{®} or any other similar product, although these do not represent a preferred embodiment because of the already mentioned drawbacks. On the contrary, in a preferred embodiment, the dECM is derived from the tissue of origin of the particular tumor type to model because it ensures the presence of ECM proteins and bioactive molecules found in the normal microenvironment of the tumor. The general process for preparing a dECM from a certain tissue is explained below. The particular procedure to prepare a dECM from the tissue of origin when this tissue is breast tissue, is explained in detail in the examples.

In step a) of the process of the invention, the bioink for producing the inner core is first prepared, the process comprising mixing at least the tumor cells with dECM. In a preferred embodiment of the invention, the dECM for bioprinting the inner core is enriched with HAMA. In case the bioink is enriched with an additive such as HAMA, it is preferred to first mix the dECM with the HAMA and then with the tumor cells.

In a particular and preferred embodiment of the process of the invention, the tumor cells used in step a) are cells derived from a patient's tumor. Further particular embodiments are those where cells are selected from different subtypes of breast tumors, preferably MDA-MB-231, MCF-7 and HCC1806 cell lines; pancreatic tumors, preferably MIA Paca2 and Capan-2 cell lines; lung tumors, preferably H69PR and A549 cell lines; kidney tumors, preferably A-498 and 786-O cell lines; hepatic tumors, preferably HepG2 and HepT1 cell lines; prostate tumors, preferably DU145, PC3, and LNCaP cell lines; and ovarian tumors, preferably SK-OV-3, A2780 and OVCAR-3 cell lines. Actually, the preferred embodiment of the invention comprises the use, in step a) of the process, of breast tumor cells derived from a patient's tumor.

For those embodiments where the inner core comprises additional cells such as immune cells, the process may include the addition of further cells in the preparation of the bioink of step a) in addition to tumor cells. In a particular embodiment of the invention, step a) additionally comprises mixing human immune cells such as macrophages, natural killer cells, dendritic cells, and/or T-lymphocytes, together with the tumor cells and the dECM and optionally with the HAMA.

The preparation of the second bioink is carried out in step b) of the process of the invention. The second bioink is aimed for bioprinting the outer stromal layer and it comprises mixing the fibroblasts and/or endothelial cells and dECM.

In step b) of the process of the invention, the use of fibroblasts and endothelial cells isolated from the patient having the tumor to be tested represent a preferred embodiment of the invention, however, also commonly known and established endothelial and fibroblasts cell lines may be used. For instance, commercial human fibroblast cell lines such as breast, lung, pancreatic, hepatic, and/or prostate fibroblasts, may be used in the process of the invention. Similarly, human vein or arterial endothelial cells lines such as HUVEC and/or HAEC are suitable for the process of the invention.

As in the case of the preparation of the first bioink, for those embodiments where additional cells such as immune cells are to be introduced in the outer stromal shell, the process may include the addition of further cells in the preparation of the bioink of step b), in addition to fibroblasts and endothelial cells. In a particular embodiment of the invention, step b) additionally comprises mixing human immune cells such as macrophages, natural killer cells, dendritic cells, and/or T-lymphocytes, together with the tumor cells and the dECM and optionally with any other additive.

In addition to HAMA that is preferably included in the preparation of the bioink for printing the inner core, other additives may be included in both the first and the second bioink to modify any desired property. These additives include, but are not limited to, collagen, methacrylated gelatin, and hyaluronic acid.

Once the two bioinks are prepared, the bioprinting steps for producing the three-dimensional tumor model of the invention are then executed. The basic and general procedure for bioprinting the model of the invention comprises carrying out step c) followed by step d). This complete sequence comprises the steps of:
c) three-dimensional printing of the inner core with the first bioink, optionally followed by the irradiation with UV light when the dECM is enriched with HAMA; and
d) three-dimensional printing of the outer stromal shell with the second bioink by completely surrounding the inner core followed by incubation of the resulting tumor model at a temperature between 25 and 40 °C.

The bioprinting of the inner core may be made with any desired morphology, be it regular or irregular. In a particular embodiment, it may be printed with a regular shape, such as a sphere or spheroid. This embodiment may represent the first stages of tumorigenesis and may allow to monitor tumor progression over time.

In a still particular embodiment, the bioprinting of the inner core can be carried out with an irregular shape, as can be the situation of a more advanced tumor stage. Reproducing the exact shape of the tumor of a patient in terms of morphology and volume allows for a more realistic assessment of patient tumor evolution and progression and has the advantage of helping to design the better therapeutic approach for a specific patient's tumor.

Although, any tumor size can be reproduced, it is preferred that the size of the inner core does not exceed dimensions of 1 mm to 4 mm of maximum diameter, preferably from 1 mm to 3.5 mm of maximum diameter, more preferably 1 to 3 mm of maximum diameter.

As explained before, in a preferred embodiment the bioink used for the bioprinting of the inner core is enriched with HAMA. In such a case, after bioprinting the inner core is stabilized via UV crosslinking. Otherwise, in case no HAMA is present in the bioink of the inner core only incubation at a temperature between 25 and 40 °C, as in step d) of the process is necessary for the dECM bioink crosslinking.

As for step d), i.e., the three-dimensional printing of the outer stromal shell with the second bioink, different bioprinting procedures known to the person skilled in the art can be used, as long as it results in the complete coating of the inner core. For instance, in a particular embodiment as explained in example 4 and also represented in figure 8B, the bioprinting of the outer stromal cell is made by bioprinting four-layered spherical structures that, once compacted, completely surround the inner core. Nevertheless, any skilled person in bioprinting technology is aware of how to surround an inner core with an outer stromal shell, depending on the particular shape and dimensions of the tumor to be coated.

The dimensions of the outer stromal shell will depend on the dimensions of the inner core itself. However, since in a preferred embodiment the bioprinted inner core does not exceed dimensions of 1 mm to 4 mm of maximum diameter, the complete three-dimensional tumor model including inner core and outer shell has a volume in the range of 0.3 cm³ to 2 cm³, preferably from 0.4 cm³ to 1.6 cm³, more preferably from 0.3 cm³ to 1 cm³.

Since in the model of the invention the stromal outer shell preferably does not contain HAMA, crosslinking of the shell is simply performed by incubation at a temperature between 25 and 40 °C.

In the case, where the particular dECM used in the preparation of the first and second bioinks discussed above is not commercially purchased, the dECM can be prepared prior to the steps a) and b) of bioinks production. In such as case, it is preferred and recommended to prepare a dECM from the same tissue of origin as the tumor to be modeled. For instance, if a breast tumor model is to be prepared, it is desirable to use breast tissue to prepare the dECM.

The production of dECM bioink from a certain tissue comprises:
i. Dissecting and slicing the tissue of origin of the tumor; i.e. breast tissue;
ii. Washing the tissue resulting from step i) with saline buffer;
iii. Freezing the resulting tissue from step ii);
iv. Treating the resulting tissue from step iii) with 0.5-1% anionic surfactants; and/or bile acids, preferably for 48-92 hours at 18-25 °C;
v. Washing the resulting tissue from step iv) with water, preferably for 1-2 hours at 18-25 °C;
vi. Treating the resulting tissue from step v) with nucleases, preferably for 12-24 hours at 30-40 °C;
vii. Washing the resulting tissue from step vi) with water, preferably for 1-2 hours at 18-25 °C;
viii. Treating the resulting tissue from step vii) with organic solvents, preferably for 6-24 hours at 18-25 °C;
ix. Washing the resulting tissue from step viii) with saline buffer, preferably at 18-37 °C;
x. Sterilizing the resulting tissue from step ix);
xi. Freeze-drying and milling the resulting tissue from step x);
xii. Acidic digestion of the powder resulting from step xi); and
xiii. Neutralization of the product resulting from step xii).

In a particular embodiment of the invention, the dECM produced is from porcine origin. In another particular embodiment, the dECM is derived from breast tissue and more particularly from porcine breast tissue as disclosed in examples 1 and 3 below.

### Method for testing anticancer drugs

A further aspect of the invention is represented by a method for testing anticancer drugs comprising the step of contacting the drug to be tested with a bioprinted three-dimensional tumor model according to the invention.

This general method can be referred to throughout the present description as the "testing method of the invention" or simply as "the method of the invention".

The testing method of the invention allows assessing the response of a certain tumor model to a certain drug. The method allows the study of a therapy or a pharmacological treatment during a period of time by the direct analysis of the progression or regression of cell growth in a certain tumor model. This method is extremely useful in research, such as for designing new and innovative therapies against different types of tumorigenic diseases and especially in designing a personalized medicine against a particular patient's tumor.

As the bioprinting technology allows for a multiplicity of reproductions of a tumor model with the same characteristics, in a particular embodiment the method according to the invention is a high-throughput test wherein three-dimensional tumor model is with the same dimensions and morphology are systematically bioprinted to form an array of tumor models and the test is carried out with multiple drugs, each being contacted with a different tumor model, at the same time. This embodiment allows for simultaneously testing, in a multi-well plate, a high number of drugs to determine which are the most effective drugs against a certain tumor and/or the effects of these drugs on the tumor progression. This particular embodiment is an important step in the development of personalized therapies against a patient's tumor.

A further embodiment of the testing method of the invention is a high-throughput testing wherein several three-dimensional tumor models differing in one or more parameters such as the donor patient tumor cells or any other parameter, are tested at the same time against the same drug. This embodiment allows for simultaneously testing a single drug towards several tumor models and appears extremely useful in the research and discovery of new drugs and therapies in pharmacology.

### Use of the bioprinted three-dimensional tumor model

A final aspect of the invention, which is somehow linked to the previous one, is the use of the bioprinted three-dimensional tumor model of the invention to reproduce the physiopathological complexity of the tumor microenvironment to develop a personalized medicine and for developing new therapies based on the understanding of the tumor progression in a three-dimensional environment mimicking the tumor microenvironment.

In other words, the three-dimensional tumor model of the invention is a powerful researching tool that not only allows for real-time analysis of what happens in the progression of a certain tumor as a physiopathologic event, but also for the development of new therapies and for adapting and improving existing ones.

Having the three-dimensional tumor model that mimics the tumor microenvironment has the advantage of reproducing the natural conditions of a certain tumor *in vitro,* which represents a great step forward in cancer research.

It is also a very important step in the development of personalized therapies based on the direct *in vitro* pharmacological testing on representative models of a patient's tumor, not only with regard to the morphology and volume of the tumor but also in the microenvironment of the tumor itself.

### EXAMPLES

The following examples disclose the preparation, characterization and validation of a three-dimensional breast tumor model comprising the features of the invention. This specific tumor model is herein described only as a particular and preferred embodiment, but any similar model based on any other type of tumor may be prepared by a skilled person following the indications of the examples presented herein.

In this respect, the present examples do not intend to limit the scope of the present invention in any way.

### Example 1: Preparation of decellularized porcine breast ECM

### 1.1) Obtaining breast tissue

Porcine breast tissue was obtained from a local slaughterhouse, so the approval of any ethics committee was not required. Tissues were washed and frozen at -80 °C until use.

### 1.2) Decellularization of porcine breast tissue

Tissues were cut in 1 cm³ pieces and washed with a phosphate buffer (PBS) prior to a freeze/thawing step.

In order to effectively decellularize the tissue, tissue fractions were subjected to 0.5-1% anionic surfactants for 48-92 hours, under orbital shaking (180 rpm) at room temperature.

Thereafter, to eliminate residual reagents, washing steps were carried out between treatments with PBS or distilled water under orbital shaking (180 rpm) at room temperature.

Removal of residual DNA from tissue fractions was carried out by treatment with nucleases (40U/mL) for 12-24 hours at 37 °C in a 1.5 mM magnesium chloride buffer as an enzyme cofactor.

After rinsing the tissue resulting from the previous step, it was treated with organic solvents for 6-24 hours prior to a final PBS and distilled water washing step and a sterilizing step under orbital shaking (180 rpm) at room temperature.

The final product is obtained by freeze-drying and milling the dECM.

### 1.3) Demonstration of the decellularization

Formalin-fixed native and dECMs were included in OCT and cryo-sectioned for histological evaluation. These sections were stained by hematoxylin/eosin following own procedure consisting of alcohol re-hydration to distilled water process prior to 3 minutes hematoxylin treatment. Then, sections were immersed in warm tap water and 15 seconds of eosin treatment. Finally, tissue sections were de-hydrated with alcohol and xylene and mounted in DPX mounting media.

Re-hydrated tissue sections were also subjected to permeabilization by incubating them with 0.3% triton X-100 solution for 30 minutes, stained with 1 µg/mL DAPI for 30 minutes and mounted in Fluoromount-G.

Cryo-sections of 7 µm were hydrated in water and submerged in propylenglycol for 2 minutes. Subsequently, tissue sections were stained with Oil Red O and developed with 85% propylenglycol for 1 minute for de-lipidation evaluation. Prior to mounting in Mowiol 4-88, 2 washings with distilled water were performed.

Remnant single and double strain DNA was analyzed Quant-iT PicoGreen kit following the manufacturers' instructions and including calf thymus DNA as standard. The DNA was extracted from the digestion with proteinase K of the frozen dECMs and native tissues by ethanol precipitation. Detection was carried out in Appliskan multimode microplate reader (Thermo Scientific).

### 1.4) Results

1. Summary of the dECM ink obtention process is shown in FIG.1 where structure A corresponds to the native tissue, structure B corresponds to decellularized ECM, structure C corresponds to dECM powder, and structure D corresponds to dECM ink.
2. Histological analysis of structures 1 and 2 was performed to confirm decellularization. Referring to FIG. 2, HE staining showed the preservation of the ECM structure (A vs B), while both HE and DAPI demonstrated the absence of nuclei in the dECM (D) as compared to ECM (C). Oil Red O staining showed significant de-lipidation (E vs F).
3. All the assayed dECMs showed very low remnant DNA (<50 ng/mg tissue) meeting the decellularization criteria established by Crapo *et al.* (Crapo PM *et al.* 2011), as depicted in FIG. 2G.

### Example 2: Breast dECM ink characterization

### 2.1) Rheology of the dECM and the dECM/HAMA combination

The resulting dECM and the dECM/HAMA inks were assessed rheologically on a MCR302 rheometer equipped with either a 25 mm diameter cone and plate geometry for the uncrosslinked bioink or a profiled parallel plate for the cross-linked gels, a Peltier plate between 10 and 40 °C, and a humidity chamber to maintain the samples hydrated during the measurements. The excess material was trimmed before the measurements began.

### 2.2) SEM microscopy characterization

Cell-free dECM bioinks were cross-linked for 30 minutes at 37 °C. dECM/HAMA bioinks were crosslinked for 5 seconds under UV light. Both samples were freeze-dried before coating them with a thin gold/paladium layer by magnetron sputtering before being imaged with a JSM-6490LV microscope (Jeol) with a working distance of 10 mm and 15 kV voltage.

### 2.3) Proteomic characterization

Samples were treated following the protocol described by Wisniewski et al. [Wisniewski 2009] with minor modifications and analyzed in a hybrid trapped ion mobility spectrometry - quadrupole time of flight mass spectrometer (timsTOF Pro with PASEF, Bruker Daltonics) coupled online to a nanoElute liquid chromatograph (Bruker). The sample (200 ng) was directly loaded in a 15 cm Bruker nanoelute FIFTEEN C18 analytical column (Bruker) and resolved at 400 nL/min with a 100 min gradient. The column was heated to 50 °C using an oven. Protein identification and quantification was carried out using MaxQuant software using default settings [Cox 2008]. Searches were carried out against a database consisting of pig protein entries (Uniprot/Swissprot+TrEMBL), with precursor and fragment tolerances of 20 ppm and 0.05 Da. Only proteins identified with at least two peptides at FDR<1% were considered for further analysis.

### 2.4) Biocompatibility of dECM inks

Biocompatibility of the dECM inks was demonstrated by LDH assay Kit and Live/Dead staining with calcein and propidium iodide following manufacturers' instructions by culturing MDA-MB-231, HBF and TeloHAEC cells for 24, 48 and 72 hours in the previously crosslinked dECMs.

### 2.5) Results

1. The rheological properties of the dECM ink were assessed to define the printing conditions, as well as the choice of the printing method. The breast dECM ink presented a linear viscoelasticity range (FIG. 3A) and showed a solid-like behavior (FIG. 3B) as the storage modulus (G') was greater than the loss modulus (G") within the frequency sweep. Also, the dECM ink had a shear thinning behavior (FIG. 3C) (viscosity decreases as shear rate increases), meaning that this material could be finely printed by pneumatic extrusion. The temperature ramp demonstrated the ink starts thermal gelation at temperatures over 25 °C (FIG. 3D).
   After incubating cell-free gels for 30 minutes at 37 °C, both G' and G" increased (FIG. 3E), acquiring stable values of around 10⁴-10⁵ Pa at frequencies ranging from 10 to 500 rad/s within the linear viscoelasticity region (FIG. 3F).
2. SEM microscopy images (FIG. 4 A and B) of the dECM showed a random orientation of the fibrillar proteins in the gel, and an amorphous surface. These properties could be tuned by combining dECM with other materials.
   SEM microscopy images (FIG. 5 A) of the dECM combined with HAMA 2% (w/v) showed a porous and fibrillar amorphous structure that allows the growth and attachment of cells. The incorporation of HAMA 2% (w/v) improved the viscosity (FIG. 5B) and therefore the printability potential of the bioink, stabilizing G' and/or G" values but keeping them in the range of 10⁴-10⁵ Pa (FIG. 5C-D).
3. The proteomic characterization of the native ECM, dECM and ink revealed that the decellularization protocol partially changes the overall protein composition of the matrix, although most of the components are maintained (FIG. 6). Less peptides were identified in the ink product, as the previous digestion step may be interfering with the generation of tryptic peptides required for the LC-MS/MS technique, revealing a complex biochemical composition though.
4. The biocompatibility of the ink was demonstrated by LDH assay by culturing MDA-MB-231, human breast fibroblasts and TeloHAEC endothelial cells for 24, 48 and 72 hours with the previously crosslinked dECMs. A graph depicting the cytotoxicity (<20%) of the dECM is described in FIG 7A. Cellular viability of cells embedded in the dECM is showed in FIG 7B after Live/Dead cell kit staining with calcein (live cells, green) and propidium iodide (dead cells, red) (>80% viable cells). Cell proliferation from day 1 to day 7 in the dECM is depicted in FIG7C.The dECM met the ISO 10993-5:2009 *in vitro* cytotoxicity requirements (>70% viable cells).

### Example 3: Formulation of the dECM inks

The powder resulting from the milling in example 1 was digested in 1 mg/mL pepsin in 0.5 M acetic acid for 48 hours at constant stirring to a final concentration of 25 mg/mL. After two days, pH was neutralized and diluted to 20 mg/mL before its use.

In order to obtain the 'inner core' bioink, neutralized dECM ink was mixed with HAMA 2% (w/v) and then with human breast cancer cells (MCF-7 and MDA-MB-231 cells).

In order to obtain the 'outer stromal shell' bioink, neutralized dECM ink was mixed with human breast fibroblasts (HBF) and aortic endothelial cells (TeloHAEC).

### Example 4: Bioprinted 3D Human Breast Tumor Model with Stromal Components

### 4.1) Cell lines

Normal human breast fibroblasts (HBF) were acquired from Innoprot (Vizcaya, Spain) and cultured according to the manufacturer's instructions. TeloHAEC human aortic endothelial cells (EC) were acquired from a collaboration group in the CIC biomaGUNE center and cultured in EGM-2 endothelial cell media (Lonza, Allendale, N.J.). MCF-7 and MDA-MB-231 cells were acquired from a collaboration in the CIC biomaGUNE and cultured in DMEM complete media. Prior to bioprinting, cells were fluorescently labelled with different emission cell trackers. Cancer cells were stained with CellTracker^{™} Green CMFDA Dye, HBF with CellTracker^{™} Deep Red and EC with CellTracker^{™} Blue CMF₂HC Dye (ThermoFisher) in order to assess 3D architecture and relative positions of cell types in the construct.

### 4.2) 3D model generation

The cancer compartment of the constructs was comprised of 100% MCF-7 or MDA-MB-231 cells. The stromal compartment of the constructs was comprised of 50% HBF and 50% TeloHAEC. For the tumor compartment, cancer cells were mixed in the mammary dECM bioink (2%) enriched with HAMA (2%) at a concentration of 15 million cells/mL. Tumor cores were printed as 4-layered circular structures with a dimension of 3 mm of diameter, with a 3DDiscovery Evolution printer (regenHU) equipped with a biosafety cabinet. Immediately following bioprinting, core constructs were stabilized via UV crosslinking. For the stromal compartment, HBF and EC were mixed in the appropriate proportions in the mammary dECM ink at a concentration of 10 million cells/mL. Stromal compartment was printed surrounding the tumor core compartment as 4-layered circular structures with a dimension of 1 cm × 1 cm × 4 mm. Immediately following bioprinting, tumor constructs were positioned at a temperature of 37 °C in the incubator to allow dECM bioink crosslinking. After 10 minutes, complete DMEM culture media was added to the culture wells. Constructs were then incubated for up to 7 to 14 additional days.

### 4.3) 3D model imaging

All confocal images were taken using a Zeiss LSM 880 confocal laser scanning microscope equipped with 405 nm (blue fluorophore excitation), 488 nm (green fluorophore excitation) and 633 nm (far red fluorophore excitation), and Plan-Apochromat 10× objective (0.45 N.A.) and Plan-Apochromat 20× objective (0.8 N.A.) In the case of 3D characterization of tumor constructs, Z-stacks of approximately 50 µm in thickness were obtained and post-imaging 3-pixel median filter applied prior to 3D rendering, to obtain images from different angles.

### 4.4) Results

1. Bioprinted breast tumor cell constructs were developed by the bioprinting technology assisted generation of a cancer tumor core, composed of MCF-7 and MDA-MB-231 breast cancer cells, surrounded by a stromal compartment composed of human breast fibroblasts and TeloHAEC cells. Bio-inks were produced by combining cells with the decellularized extracellular matrix bioink. Cancer core bioink was produced by combining cancer cells with the dECM enriched in HAMA. FIG. 8A shows an illustrative example of a breast lobular carcinoma tumor microenvironment. FIG. 8B shows a schematic diagram depicting the construction of the construct. FIG. 8C shows a photograph of the tumor cells core immediately after bioprinting and crosslinking of the hydrogel. FIG. 8D shows a photograph of the construct immediately after bioprinting the stromal compartment and crosslinking of the dECM.
2. Confocal imaging analysis of 3D constructs was performed to assess tissue architecture and relative positions of cell populations. The spatial distribution of each cell population in the 3D model after bioprinting can be observed in FIG. 9A, wherein cancer cells core (green) is observed as a rounded structure that is surrounded by HBF (pink) and EC (cyan). Higher magnification images of each cell population comprising the model is also detailed in FIG. 9. All cell populations integrating the model [MDA-MB-231 cancer cells in green (FIG. 9B), HBF in pink (FIG. 9C) and EC in cyan (FIG. 9C)] displayed their expected physiological phenotype. Hematoxylin-Eosin staining images of the stroma and the core of the model are also depicted in FIG 9D. To assess construct to construct reproducibility, confocal images of different tumor core constructs and 3D breast cancer models are presented in FIG. 10.

### Example 5: Bioprinted 3D constructs cell viability

### 5.1.) Cell viability assessment

To assess cell viability in both the core tumor model and the 3D breast tumor model with the stromal compartment, calcein (targets live cells in green) and propidium iodide (targets dead cells in red) were added to the cell culture medium of the 3D constructs at a 1:100 concentration.

### 5.2) Results

1. Cell viability in the 3D constructs is shown in FIG. 11 and FIG. 12. FIG. 11 shows cell viability of the bioprinted tumor core over time. Live/Dead staining showed that bioprinted tumor core structures developed a central necrotic core (red dead cells), which is maintained over time (Days 1-7). This necrotic core reveals cell death due the lack of nutrients and oxygen that arrive to the inner cells of the construct. This oxygen and nutrient gradient with the subsequent creation of a tumor necrotic core closely resemble the nature of patient's tumors. Cell viability in the 3D tumor model comprising the stromal compartment is depicted in FIG. 12. The model shows high cell density and cell viability on day 7 after printing (high % of green cells).

### Example 6: Bioprinted 3D tumor model microenvironment activation characterization

### 6.1) Immunofluorescence assays

3D tumor constructs were fixed in 4% paraformaldehyde solution. Constructs were then permeabilized with TBS-Tween20 1% solution for 30 minutes and then blocked with 1% BSA in PBS solution for 2 hours, followed by incubation with respective primary antibodies (1:50) at 4 °C for 3 consecutive days. Constructs were then washed three times in TBS with 0.1% Tween 20 and incubated with appropriate AlexaFluor-conjugated secondary antibodies diluted 1:500 in TBS. Constructs were then washed three times in TBS-0.1% Tween 20 and DAPI staining (1:500) was added. Confocal 3D imaging was then performed.

### 6.2) Cytokine array

Acellular dECM, 3D core and 3D tumor constructs culture supernatant were collected at day 7 and were prepared to perform a Proteome Profiler Human XL Cytokine Array following manufacturers' instructions. The resulting images were analyzed with imaged software.

### 6.3) Results

1. Immunofluorescence assays were carried out to characterize the ability of the constructs to reproduce the creation of a pathological tumor microenvironment. Staining for cell-type specific markers revealed the activation of biomarkers that are pathologically expressed in tumors. FIG. 13 shows that HBF express Vimentin and α-SMA biomarkers, revealing the transition towards a malignant cancer associated fibroblasts phenotype in the 3D model. Referring to FIG. 14, the staining with tumoral biomarkers SLUG, TWIST 2 and TGF-β reveal that the pathological activation of these pathways can be reproduced in the 3D tumor construct. FIG.15 shows the formation of EC aggregations (CD31+ cells) that can lead to the angiogenic process in the 3D tumor model.
2. FIG. 16 shows the results of the cytokine array assay. This assay showed that the 3D tumor model induces the expression of specific cytokines related to tumor immunosuppression, tumor proliferation, ECM remodeling and cancer stem cells populations reflecting the ability of the model to reproduce intrinsic features of the tumor biology.

### Example 7: Modeling Drug Responses in Human Breast Cancer Tumor Models with Stromal Components

### 7.1) Drug testing assays

3D bioprinted breast cancer tumor models of example 4 [constructed with stromal (50% HBF,50% TeloHAEC in mammary dECM) and either tumor MCF-7 or MDA-MB-231 cells embedded in the HAMA-enriched mammary dECM] were sequentially bioprinted in a 48-well high-throughput testing plate (FIG. 17A) and then exposed to increasing concentrations of Tamoxifen (see FIG. 17B), Paclitaxel (see FIG. 17C), or Doxorubicin (see FIG. 17D) for 3 days. Cytotoxicity was measured using lactate dehydrogenase (LDH) assay. Data shown is the summary of a minimum of three biological replicates in three independent experiments.

### 7.2) Results

1.The MCF-7 and MDA-MB-231 breast cancer tumor models were and exposed to specific antitumor compounds to assess drug response. Referring to FIG. 17A, multiple bioprinted breast cancer tumor models were treated with media alone (cytotoxicity negative control), Triton X-100 (cytotoxicity positive control), or increasing concentrations of tamoxifen, paclitaxel and doxorubicin for 48 hours and assessed for cytotoxicity by LDH release assay. For each graph, the % of cytotoxicity indicates the cytotoxicity for each concentration compared to negative and positive controls.
2. Regarding FIG. 17 B, MCF-7 3D tumor models were more susceptible to tamoxifen-induced toxicity than MDA-MB-231 cells 3D tumor models when treated with the same dose of tamoxifen for the same duration. Tamoxifen is a therapeutic compound that specifically targets estrogen receptor positive tumors (MCF-7 model) while it cannot impair the proliferation of estrogen receptor negative tumors (MDA-MB-231 model); hence, these results indicate that developed 3D tumor models can reflect the therapeutic response observed in the clinics. Referring to FIG. 17C and FIG. 17D, it was found that both Paclitaxel and Doxorubicin (no directed therapy) had a similar effect in cytotoxicity in both 3D tumor models. FIG. 16E shows live/dead staining of MCF-7 tumor models treated with 200 µM Tamoxifen compared to negative and positive controls. Dead cells are observed in the outer regions of the models, corresponding to a higher drug exposure of those cell populations compared to the ones situated in the center of the model. Overall, developed 3D tumor models can be used as preclinical drug testing platforms.

## Claims

1. A bioprinted three-dimensional tumor model comprising:
a) An inner core comprising tumor cells; and
b) an outer stromal shell comprising fibroblasts and/or endothelial cells surrounding the core;
wherein the cells from the core and the stromal shell are embedded in a decellularized extracellular matrix (dECM).

2. The tumor model according to claim 1 wherein the tumor cells are cells derived from a patient's tumor or selected from the group consisting of cell subtypes derived from breast tumors, pancreatic tumors, lung tumors, kidney tumors, hepatic tumors, prostate tumors, and ovarian tumors.

3. The tumor model according to claim 2 wherein the cell subtypes derived from breast tumors are MDA-MB-231, MCF-7 and HCC1806 cell lines; the cell subtypes derived from pancreatic tumors are MIA Paca2 and Capan-2 cell lines; the cell subtypes derived from lung tumors are H69PR and A549 cell lines; the cell subtypes derived from kidney tumors are A-498 and 786-O cell lines; the cell subtypes derived from hepatic tumors are HepG2 and HepT1 cell lines; the cell subtypes derived from prostate tumors are DU145, PC3, and LNCaP cell lines and the cell subtypes derived from ovarian tumors are SK-OV-3, A2780 and OVCAR-3 cell lines.

4. The tumor model according to claim 1 to 3 wherein the dECM is derived from the tissue of origin of the specific designed tumor model, and it is preferably from porcine origin.

5. The tumor model according to any of the previous claims wherein the dECM is derived from a healthy tissue of origin and is absent of tumor-derived factors.

6. The tumor model according to any of the previous claims wherein the dECM used for the bioprinting of the inner core is enriched with methacrylated hyaluronic acid (HAMA).

7. The tumor model according to any of the previous claims wherein the inner core has a regular or irregular morphology and wherein the inner core has a maximum diameter in the range of 1 to 4 mm and the complete three-dimensional tumor model has a volume in the range of 6 mm³ to 1 cm³.

8. The tumor model according to any of the previous claims wherein the inner core or the outer stromal shell may additionally contain immune cells and the outer stromal shell may contain other tissue specific stromal cell types including pericytes, adipocytes and/or mesenchymal cells.

9. A process to prepare a three-dimensional tumor model according to claim 1 comprising:
a) the preparation of a first bioink comprising mixing at least the tumor cells with dECM, optionally enriched in HAMA;
b) the preparation of a second bioink comprising mixing at least the fibroblasts and/or endothelial cells and dECM;
c) the three-dimensional printing of the inner core using the first bioink, optionally followed by irradiation with UV light when the dECM is enriched in HAMA; and
d) the three-dimensional printing of the outer stromal shell using the second bioink by completely surrounding the inner core followed by incubation of the resulting tumor model at a temperature between 25 and 40 °C.

10. The process according to claim 9 comprising a previous step of producing the dECM used for preparing the first and the second bioinks.

11. The process according to claim 10 where the production of the dECM comprises:
i. Dissecting and slicing the tissue of origin of the tumor;
ii. Washing the tissue resulting from step i) with saline buffer;
iii. Freezing the resulting tissue from step ii);
iv. Treating the resulting tissue from step iii) with 0.5-1% anionic surfactants; and/or bile acids;
v. Washing the resulting tissue from step iv) with water;
vi. Treating the resulting tissue from step v) with nucleases;
vii. Washing the resulting tissue from step vi) with water;
viii. Treating the resulting tissue from step vii) with organic solvents;
ix. Washing the resulting tissue from step viii) with saline buffer;
x. Sterilizing the resulting tissue from step ix);
xi. Freeze-drying and milling the resulting tissue from step x).
xii. Acidic digestion of the powder resulting from step xi).
xiii. Neutralization of the product resulting from step xii).

12. A method for testing anticancer drugs comprising the step of putting the drug to be tested in contact with a bioprinted three-dimensional tumor model according to any of claims 1 to 8.

13. The method according to claim 12 which is a high-throughput testing wherein the three-dimensional tumor model is systematically bioprinted with the same dimensions and morphology and the testing is carried out vis-à-vis multiple drugs at the same time.

14. The method according to claim 12 which is a high-throughput testing wherein several three-dimensional tumor models are tested at the same time vis-à-vis a drug.

15. Use of the bioprinted three-dimensional tumor model according to any of claims 1 to 8 to reproduce the cellular heterogeneity, the spatial architecture and the physiopathological complexity of the tumor microenvironment to develop a personalized medicine and for developing new therapies based on the understanding of the tumor progression in a three-dimensional environment mimicking the tumor microenvironment.
